Europäisches Patentamt

European Patent Office

Office européen des brevets

Veröffentlichungsnummer: **0 292 678**

**A1**

# EUROPÄISCHE PATENTANMELDUNG

Anmeldenummer: 88105614.7

Int. Cl.⁴ **A61B 17/16**

Anmeldetag: 08.04.88

Priorität: 29.05.87 DE 3718138

Veröffentlichungstag der Anmeldung:
30.11.88 Patentblatt 88/48

Benannte Vertragsstaaten:
CH FR GB LI

Anmelder: **Kernforschungszentrum Karlsruhe GmbH**
**Weberstrasse 5 Postfach 3640**
**D-7500 Karlsruhe 1(DE)**

Erfinder: **Mattheck, Claus, Dr.**
**Siemensstrasse 6**
**D-6729 Leimersheim(DE)**
Erfinder: **Börner, Martin, Dr.**
**Höchster Strasse 14d**
**D-6321 Schwalbach(DE)**

## Hilfsmittel zur Anbringung eines Kreuzbandersatzes.

Die Erfindung betrifft ein Hilfsmittel zur Anbringung eines Kreuzbandersatzes zwischen Femur und Tibia des menschlichen Kniegelenkes, wobei der Kreuzbandersatz zwischen die Rollhügel des Femur geführt wird und Schnitte durch die dorsalen Rollhügel einem Kreis nachgebildet sind, dessen fiktiver Kreismittelpunkt zwecks Anbringung einer Bohrung durch den Femur aufzusuchen ist.

Die der Erfindung zugrundeliegende Aufgabe besteht darin, ein Hilfsmittel der e. g. Art zu bieten, mit dem eine transcondyläre Bohrung individuell an den Gelenk- und Knochenbau des Patienten angepaßt optimal durchgeführt werden kann.

Die Lösung ist gekennzeichnet, durch ein Zielgerät mit zwei miteinander korrespondierenden Kontaktspießen die an jeweils einem der dorsalen Rollhügel in Berührungspunkten bei gebeugtem Knie derart anlegbar sind, daß sie senkrecht auf der durch diese Berührungspunkte verlaufenden fiktiven Kreisebene stehen, und durch eine mit den Kontaktspießen ebenfalls korrespondierende Bohrlehre zur Festlegung des Kreismittelpunktes.

Fig. 2

EP 0 292 678 A1

## Hilfsmittel zur Anbringung eines Kreuzbandersatzes

Die Erfindung betrifft ein Hilfsmittel zur Anbringung eines Kreuzbandersatzes zwischen Femur und Tibia des menschlichen Kniegelenkes, wobei der Kreuzbandersatz zwischen die Rollhügeln des Femur geführt wird und Schnitte durch die dorsalen Rollhügel einem Kreis nachgebildet sind, dessen Kreismittelpunkt zwecks Anbringung einer Bohrung durch den Femur aufzusuchen ist.

Die operative Befestigung des Transplantats zum Ersatz des vorderen Kreuzbandes am menschlichen Kniegelenk erfolgt z. B. nach der Zügelmethode gemäß Blauth. Hierzu ist eine transcondyläre Bohrung mit einem Bohrgerät durchzuführen. Verwendung findet eine Bohrlehre (DE-OS 33 12 250), die einen Bohrschlitten aufweist, an dem sowohl ein Kondylenfühler mit Zielmarkierung für einen Bohrer als auch eine Bohrhülsen-Aufnahme für den Bohrer befestigt sind. Zwar wird der Kondylenfühler zwischen den beiden Rollhügeln des Femur hindurchgeführt, die Zielmarkierung für den Bohrer und somit die Bohrung durch den Femurknochen liegt jedoch recht empirisch und von Patient zu Patient aufgrund dessen Knochenbaus recht verschieden. Die Anbringung der Femurbohrung erfolgt somit ziemlich willkürlich, da die Bohrlehren nur in bestimmten Grenzen individuell anpaßbar sind.

Aufgrund experimenteller und mathematisch ermittelter Erkenntnisse hat sich gezeigt, daß die Rollhügel des Femur, zumindest im hinteren Bereich bei allen noch so individuell gearteten Formen, jeweils Kreisbögen bilden, die in der Gelenkebene liegen. Somit ergibt sich eine optimale Führung durch den Kreuzbandersatz immer dann, wenn der Anknüpfpunkt auf der Achse liegt, die als Mittelsenkrechte auf dem hinteren Rollhügelkreis steht. Die Schwierigkeit besteht im Prinzip nur noch darin, bei der Operation vor Ort möglichst mit einfachen Mitteln diesen Krümmungsmittelpunkt aufzufinden, um eine Zielbohrung anzusetzen.

Die der Erfindung zugrundeliegende Aufgabe besteht darin, ein Hilfsmittel der e. g. Art zu bieten, mit dem eine transcondyläre Bohrung individuell an den Gelenk- und Knochenbau des Patienten angepaßt optimal durchgeführt werden kann.

Die Lösung ist in den kennzeichenden Merkmalen des Anspruches 1 beschrieben.

Die übrigen Ansprüche geben vorteilhafte Weiterbildungen und Ausführungsformen der Erfindung an.

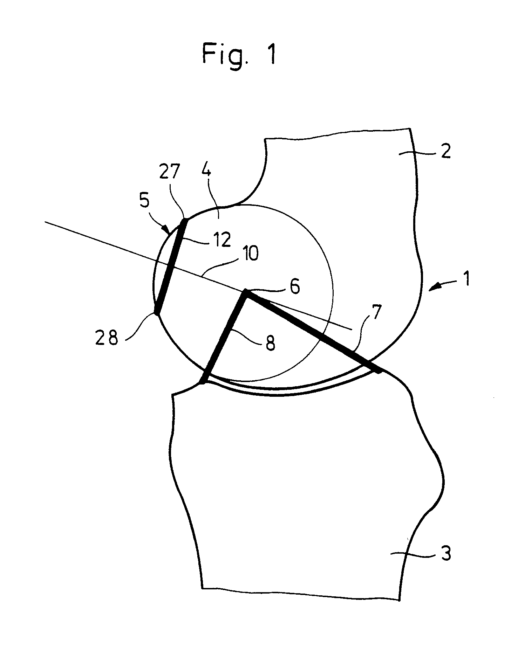

Die Erfindung wird im folgenden anhand eines Ausführungsbeispiels mittels der Figuren 1 - 3 näher erläutert.

Die Fig. 1 zeigt im Prinzip die Seitenansicht eines menschlichen Kniegelenks 1 mit dem Femur 2 und der Tibia 3. Jeder der beiden an der Rückseite des Femur 2 vorhandenen hinteren Rollhügel 4, über die das Kniegelenk 1 bei der Beugung abrollt, ist ein Kreisbogen 5, der zeichnerisch ergänzt dargestellt ist. Der Krümmungsmittelpunkt 6 ist zugleich Mittelpunkt dieses Kreises. Die optimale Führung sowohl des vorderen als auch des hinteren schematisch dargestellten Kreuzbandersatzes 7 bzw. 8 gelingt dann, wenn ihre femurale Anbindung auf einer Achse liegt, die senkrecht auf dem Kreis steht und durch den Mittelpunkt 6 geht.

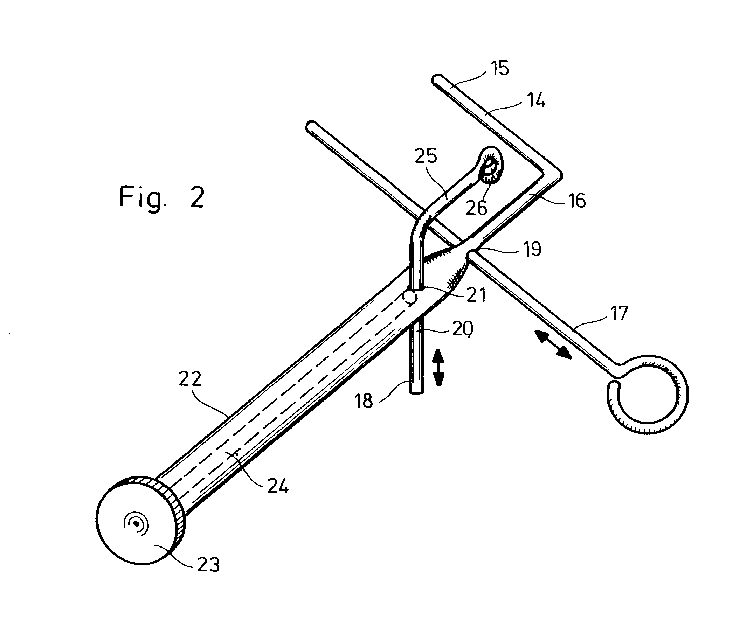

Das Hilfsmittel zum Auffinden dieses fiktiven Kreismittelpunktes, der auch notwendig ist für das Auffinden des Ansatzpunktes für die Bohrung durch den Femurknochen, ist in Fig. 2 dargestellt. Es besteht im wesentlichen aus einem rechtwinkligen Haken 14 mit zwei im Winkel zueinander ausgerichteten Hakenteilen 15, 16, einem Verriegelungsstift 17 sowie einer Bohrlehre 18.

Der Verriegelungsstift 17 und der Hakenteil 15 verlaufen parallel zueinander. Hierzu kann der Verriegelungsstift 17 in einer Bohrung 19 verstellt werden, die im zweiten Hakenteil 16 angebracht ist. Die Bohrlehre 18 besteht ebenfalls aus einem gebogenen Stab, dessen eines Stabteil 20 in einer weiteren Bohrung 21 im Hakenteil 16 bzw. im daran anschliessenden Griff 22 senkrecht zur zwischen dem Hakenteil 15 und dem Verriegelungsstift 17 aufgespannten fiktiven Ebene höhenverstellbar geführt und fixiert wird. Als Fixiermittel dient ein koaxial innerhalb des Griffes 22 mittels einer Rändelmutter 23 verstellbarer Gewindestift 24.

Das andere Stabteil 25 der Bohrlehre 18 weist eine Bohröse 26 auf, die gegenüber dem Hakenteil 15 und dem Verriegelungsstift 17 so ausgerichtet wird, daß durch sie der (nicht gezeigte) Bohrer durch den Kreismittelpunkt der dorsalen Rollhügel gezielt hindurchgeführt werden kann. Als Hilfsmethode zur Feststellung der Höhe und damit Lage der Bohröse 26 dienen seitliche Röntgenbilder vom Kniegelenk, insbesondere des Femurknochens.

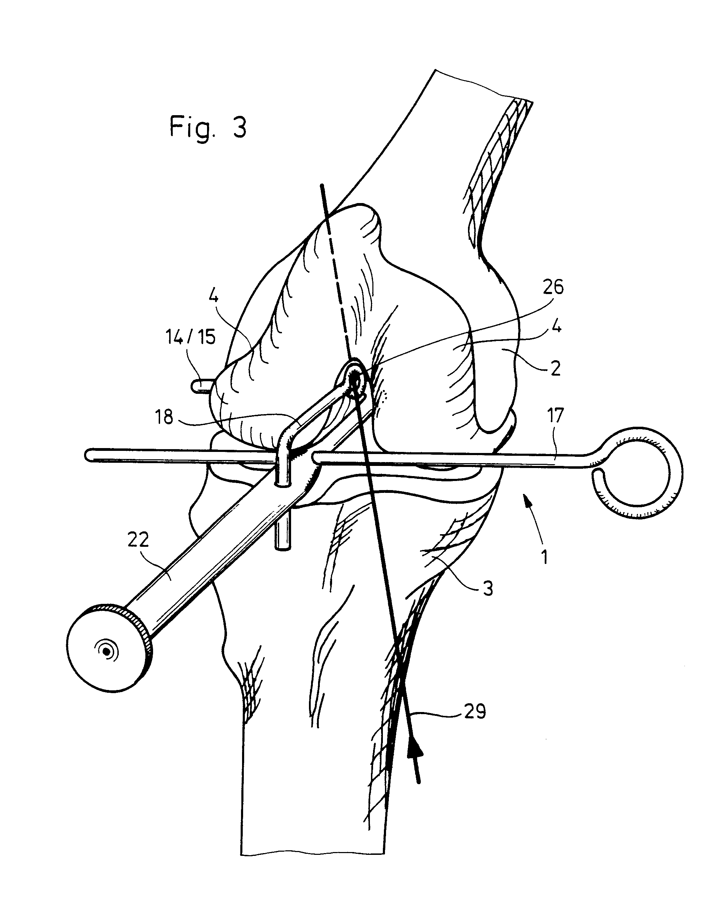

Die Anordnung des Hilfsmittels gemäß Fig. 2 am Kniegelenk 1 (gemäß Fig. 1) ist in Fig. 3 dargestellt. Zwischen den beiden Rollhügeln 4 wird der Haken 14 (15, 16) hindurchgeführt, derart, daß sein Hakenteil 15 einen Berührungspunkt mit dem im dorsalen Rollhügel verlaufenden Kreisbogen hat (z. B. Punkt 27 am Kreisbogen 5 nach Fig. 1).

Der Verriegelungsstift 17 wird so geführt, daß er ebenfalls einen Berührungspunkt (-bereich) mit diesem Kreisbogen hat (z. B. Punkt 28 nach Fig. 1). Die Bohröse 26 ist weiterhin so ausgerichtet, daß durch sie die dargestellte Bohrachse 29 gezielt durch den Femurknochen 2 hindurchverlegt werden kann. Die Bohröse 26 wird längs der Mittelsenkrechten 10 der Sehne 12 zwischen den Punkten 27. 28 zur Einjustierung auf den Mittelpunkt verschoben.

## Ansprüche

1. Hilfsmittel zur Anbringung eines Kreuzbandersatzes zwischen Femur und Tibia des menschliechen Kniegelenkes. wobei der Kreuzbandersatz zwischen die Rollhügeln des Femur geführt wird und Schnitte durch die dorsalen Rollhügel einem Kreis nachgebildet sind. dessen fiktiver Kreismittelpunkt zwecks Anbringung einer Bohrung durch den Femur aufzusuchen ist. gekennzeichnet, durch ein Zielgerät (14. 17. 18) mit zwei miteinander korrespondierenden Kontaktspießen (14. 17) die in jeweils einem der dorsalen Rollhügel (4) in Berührungspunkten (27. 28) bei gebeugtem Knie derart anlegbar sind. daß sie senkrecht auf der durch diese Berührungspunkte (27. 28) verlaufenden fiktiven Kreisebene (5) stehen. und durch eine mit den Kontaktspießen (14, 17) ebenfalls korrespondierende Bohrlehre (18) zur Festlegung des Kreismittelpunktes (6).

2. Hilfsmittels nach Anspruch 1. dadurch gekennzeichnet. daß das Zielgerät (14. 17. 18) aus einem Haken (14. 15. 16) und einem Verriegelungsstift (17) als Kontaktspieße besteht. wobei der Verriegelungsstift (17) am Hakenteil (16) gehaltert ist. und daß am Hakenteil (16) auch die Bohrlehre (18) befestigt ist.

3. Hilfsmittel nach Anspruch 1 und 2. dadurch gekennzeichnet. daß der Haken (14) an einem Ende (16) zu einem Griff (22) ausgebildet ist. in dem eine Bohrung (21) vorgesehen ist. in der die Bohrlehre (18: 20) senkrecht bezüglich einer durch die beiden Kontaktspieße (14, 17) gebildeten Ebene höhenverstellbar ist.

4. Hilfsmittel nach Anspruch 1 - 3. dadurch gekennzeichnet. daß die Bohrlehre (18) aus einem etwa rechtwinkligen Stab (20. 25) mit an einem Stabende (25) angeordneter Bohröse (26) besteht. und daß das zweite Stabende (20) durch die Bohrung (21) verläuft.

5. Hilfsmittel nach Anspruch 1 oder einem der folgenden. dadurch gekennzeichnet. daß im Griff (22) koaxial ein Gewindestift (23) verläuft. mit dem die Bohrlehre (18) fixierbar ist.

6. Hilfsmittel nach Anspruch 1 oder einem der folgenden. dadurch gekennzeichnet. daß der Verriegelungsstift (17) in einer weiteren Bohrung (19) im Hakenteil (16) haltbar ist.

## Fig. 1

Fig. 2

0 292 678

PLA 8737

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 162 027 (M. ODENSTEN et al.)<br>* Seite 4, Zeile 20 - Seite 5, Zeile 2; Figur 1 *<br>--- | 1 | A 61 B 17/16 |
| A | EP-A-0 153 831 (B. SEEDHOM)<br>* Seite 11, Zeilen 1-14; Figuren 5,6 *<br>--- | 1 | |
| A | DE-A-3 411 891 (G. DAWIDOWSKI)<br>* Figuren 1,2; Seite 6, Zeile 1 - Seite 7, Zeile 14 *<br>--- | 1 | |
| A,D | DE-A-3 312 250 (W. BLAUTH)<br>* Zusammenfassung; Figuren 1-3 *<br>----- | | |

RECHERCHIERTE SACHGEBIETE (Int. Cl.4)

A 61 B
A 61 F

Der vorliegende Recherchenbericht wurde für alle Patentansprüche erstellt

| Recherchenort | Abschlußdatum der Recherche | Prüfer |
|---|---|---|
| DEN HAAG | 30-08-1988 | NEILL M.C. |